# EUROPEAN PATENT SPECIFICATION

(11) **EP 4 658 172 B1**
(45) Date of publication and mention of the grant of the patent: **28.10.2026**
(21) Application number: 24709937.7
(22) Date of filing: 29.01.2024
(51) Int. Cl.: A61B 5/259, A61B 5/291, A61B 5/00

(54) **SENSOR WITH ELECTRODES HAVING GEL CONTAINMENT FEATURES**
SENSOR MIT ELEKTRODEN MIT GELEINSCHLUSSMERKMALEN
CAPTEUR À ÉLECTRODES AYANT DES CARACTÉRISTIQUES DE CONFINEMENT DE GEL

(30) Priority: 30.01.2023 US 202363482279 P
(43) Date of publication of application: 10.12.2025
(73) Proprietor: Becton, Dickinson and Company, Franklin Lakes, NJ 07147-1880 (US)
(72) Inventor: DALENE, Matthew, Franklin Lakes, NJ 07417 (US); SKYRUS, Oma, Franklin Lakes, NJ 07417 (US)
(74) Representative: Venner, Julia Ann
(86) International application number: PCT/US2024/013351
(87) International publication number: WO 2024/163346

(56) References cited:
- WO-A1-96/11631
- US-A1- 2002 183 605
- US-A1- 2019 223 801
- US-A1- 2023 240 581

## Description

### BACKGROUND OF THE INVENTION

### 1. Technical Field

The present disclosure relates to medical devices that use sensors in general, and to medical device that use sensors that include electrodes in particular.

### 2. Background Information

A number of different medical sensing methods utilize measurements of electrical activity. Non-limiting examples of such methods include electroencephalography ("EEG"), electrocardiograms ("ECG" or "EKG"), and electromyography ("EMG"). These methods typically use one or more sensors configured to be disposed on a patient's skin surface. Each of these sensors include at least one electrode for sensing the electrical activity. An EEG, for example, is a non-invasive method used to sense and record electrical activity of the brain. EEGs are configured to be disposed on a patient's cranial skin surface and may be used in a variety of different applications including estimating depth of anesthesia (DoA), and for diagnosing disease such as epilepsy, stroke, and encephalitis to name a few.

It is important that the electrode or electrodes have desirable electrical connectivity to the patient's skin surface to maximize signal quality. Sensors may be configured with dry electrodes that operate capacitively or that penetrate the patient's skin. Gel sensors are configured with an electrolytic gel disposed at the interface between the electrode and the skin to establish an electrically conductive path there between. The gel sensor electrodes work best when the gel provides a continuous electrical path between the electrode and the skin. Many existing sensor designs are prone to gel leakage when the sensor is applied to the skin surface with pressure; i.e., the gel is initially disposed in a fixed volume (e.g., a defined volume pocket with no inlet/exit) and compression of the fixed volume forces the gel out of the fixed volume. This leakage is problematic for several reasons. For example, if the amount of gel remaining in the fixed volume is insufficient to provide the continuous gel body between the electrode and the skin, the electrical path may be compromised. Many sensors are attached to a patient's skin using an adhesive disposed around the periphery of the electrodes. If the sensor is applied to the skin surface with pressure and gel is forced out of its initial fixed volume, the leaked gel will likely engage with and compromise the sensor adhesive. Furthermore, leaked gel can create a messy region on the patient's skin.

What is needed is a gel sensor that contains the electrolytic gel in a manner that overcomes the shortcomings of existing gel sensors.

US 2002/183605 describes an array of electrodes constructed to allow the user to easily adjust to the correct size of the patient's head. The array is self-adhesive, pre-gelled and disposable. The array fits easily over the temple and forehead areas where EEG signals can be acquired by specially designed monitors for purposes of monitoring a number of bodily phenomena, including but not limited to, depth of anesthesia, and/or ischemia, and burst suppression. The array is connected to the monitor via a tab connector that is integral to the disposable device. The tab connector is insertible into a reusable connector that is part of a monitoring system.

### SUMMARY

The claimed invention is set out in the independent claim. Particular embodiments of this aspect are set out in the dependent claims. Any subject matter contained herein that does not fall within the scope of the appended claims is considered as being useful for understanding the invention.

According to an aspect of the present disclosure, a physiological sensor assembly is provided that includes at least one electrode having a sensing surface and a body. The body has a plurality of exterior surfaces including a contact surface, at least one pocket disposed in the contact surface, at least one gel vent in fluid communication with the at least one pocket, and at least one vent passage configured to provide fluid communication between the at least one gel vent and at least one exterior surface. The at least one pocket is open to the contact surface. The at least one electrode is disposed in the at least one pocket.

In any of the aspects or embodiments described above and herein, the sensor assembly may include an electrolytic gel disposed in the at least one pocket in contact with the sensing surface of the at least one electrode.

In any of the aspects or embodiments described above and herein, the at least one gel vent may be configured to receive electrolytic gel extruded from the at least one pocket, and the at least one vent passage may be configured to permit air to escape from the at least one gel vent.

In any of the aspects or embodiments described above and herein, the at least one vent passage may be configured to provide fluid communication between the at least one gel vent and the contact surface.

In any of the aspects or embodiments described above and herein, the body exterior surfaces may include a back surface disposed opposite the contact surface, and the at least one vent passage may be configured to provide fluid communication between the at least one gel vent and the back surface.

In any of the aspects or embodiments described above and herein, the body exterior surfaces further may include a back surface disposed opposite the contact surface, and at least one side surface extending between the back surface and the contact surface around a periphery of the body, and the at least one vent passage may be configured to provide fluid communication between the at least one gel vent and the at least one side surface.

In any of the aspects or embodiments described above and herein, the at least one pocket may be configured to provide a void around at least a portion of a periphery of the at least one electrode, and the at least one gel vent may be in fluid communication with the at least a portion of the periphery of the at least one electrode.

In any of the aspects or embodiments described above and herein, the at least one pocket may be configured to be substantially contiguous with a periphery of the electrode, and the at least one gel vent may be in fluid communication with a portion of the pocket disposed between the sensing surface of the at least one electrode and the contact surface.

In any of the aspects or embodiments described above and herein, the at least one pocket may include a first pocket and a second pocket, and the at least one gel vent may include a first gel vent in fluid communication with the first pocket and a second gel vent in fluid communication with the second pocket, and the first gel vent may be independent of the second gel vent.

In any of the aspects or embodiments described above and herein, the at least one pocket may include a first pocket and a second pocket, and the at least one gel vent may include a first gel vent in fluid communication with the first pocket and a second gel vent in fluid communication with the second pocket, and the first gel vent may be in fluid communication with the second gel vent.

In any of the aspects or embodiments described above and herein, the at least one pocket may include a first pocket and a second pocket, and the at least one gel vent may include a first gel vent in fluid communication with the first pocket and a second gel vent in fluid communication with the second pocket, and the sensor may further include a common gel vent portion in fluid communication with the first gel vent and the second gel vent, and the at least one vent passage may provide fluid communication between the common gel vent portion and the at least one exterior surface.

In any of the aspects or embodiments described above and herein, the at least one gel vent and the at least one vent passage may be enclosed within the body.

In any of the aspects or embodiments described above and herein, the at least one pocket may include a first pocket, and the at least one gel vent may include a first gel vent and a second gel vent, and the at least one vent passage may include a first vent passage configured to provide fluid communication between the first gel vent and the at least one exterior surface, and a second vent passage configured to provide fluid communication between the second gel vent and the at least one exterior surface.

In any of the aspects or embodiments described above and herein, the at least one exterior surface may include a first exterior surface and a second exterior surface, and the first vent passage may be configured to provide fluid communication between the first gel vent and the first exterior surface, and the second vent passage may be configured to provide fluid communication between the second gel vent and the second exterior surface.

In any of the aspects or embodiments described above and herein, the body may include a main body portion and a discrete body portion connected to one another by a flexible connector configured to provide electrical communication between the main body portion and the discrete body portion, and the at least one pocket may include a first pocket and a second pocket disposed in the contact surface of the main body portion and a third pocket disposed in the discrete body portion, and the at least one gel vent may include a first gel vent in fluid communication with the first pocket, a second gel vent in fluid communication with the second pocket, and a third gel vent in fluid communication with the third pocket.

In any of the aspects or embodiments described above and herein, the sensor may be configured as an electroencephalography ("EEG") sensor.

According to an aspect of the present disclosure, a system for sensing a physiological parameter is provided that includes at least one sensor assembly and a base unit. The at least one sensor assembly includes at least one electrode and a body. The at least one electrode has a sensing surface, and the electrode is configured to produce first electrical signals representative of second electrical signals sensed from a patient. The body has a plurality of exterior surfaces including a contact surface, at least one pocket disposed in the contact surface, at least one gel vent, and at least one vent passage. The at least one pocket is disposed in the contact surface, and is open to the contact surface and the at least one electrode is disposed in the at least one pocket. The at least one gel vent is in fluid communication with the at least one pocket. The at least one vent passage is configured to provide fluid communication between the at least one gel vent and at least one said exterior surface. The base unit has a system controller in communication with the at least one electrode of the sensor assembly and a non-transitory memory storing instructions, which instructions when executed cause the system controller to process the first electrical signals to produce information relating to the physiological parameter.

In any of the aspects or embodiments described above and herein, the at least one sensor assembly may be configured as an electroencephalography ("EEG") sensor and the information relating to the physiological parameter may include EEG information.

The foregoing features and elements may be combined in various combinations without exclusivity, unless expressly indicated otherwise. These features and elements as well as the operation thereof will become more apparent in light of the following description and the accompanying drawings. It should be understood, however, the following description and drawings are intended to be exemplary in nature and non-limiting.

### BRIEF DESCRIPTION OF THE DRAWINGS

FIG. 1 is a perspective view of a sensor assembly.
FIG. 2 is a diagrammatic planar view of a sensor assembly embodiment.
FIG. 3A is a diagrammatic planar view of a sensor main body portion embodiment.
FIG. 3B is a diagrammatic sectional view of the sensor main body portion embodiment shown in FIG. 3A.
FIG. 3C is a diagrammatic sectional view of a sensor discrete body portion embodiment.
FIG. 4 is a diagrammatic planar view of a sensor main body portion embodiment.
FIG. 5 is a diagrammatic sectional view of the sensor main body portion embodiment.
FIG. 6 is a diagrammatic planar view of a sensor main body portion embodiment.
FIG. 7 is a diagrammatic planar view of a sensor main body portion embodiment.
FIG. 8 is a diagrammatic planar view of a sensor main body portion embodiment.
FIG. 9 is a diagrammatic planar view of a sensor main body portion embodiment.
FIG. 10 is a diagrammatic planar view of a sensor main body portion embodiment.
FIG. 10A is a diagrammatic sectional view of the sensor main body portion embodiment shown in FIG. 10A.
FIG. 11 is a diagrammatic sectional view of the sensor embodiment.
FIG. 11A is a diagrammatic sectional view of the sensor embodiment.

### DETAILED DESCRIPTION

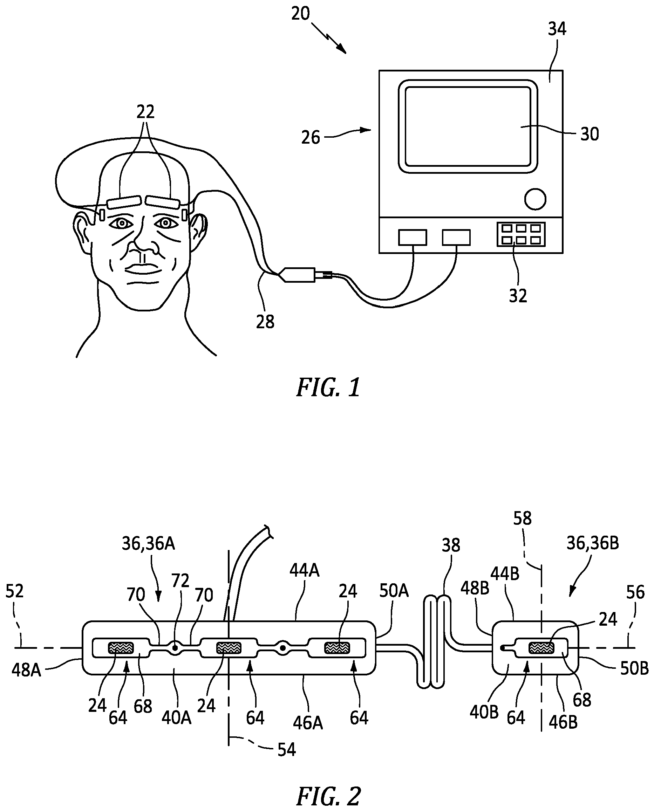

FIG. 1 is a diagrammatic illustration of a present disclosure system 20 having at least one sensor assembly 22 (two are shown) that includes one or more electrodes 24 for sensing the electrical activity of a patient. The present disclosure is described below in terms of an electroencephalograph (EEG) that may be a dedicated EEG device or may be an EEG element of a system that includes EEG sensing. An example of a system that may include EEG and other physiological sensing is one that can be used to determine depth of anesthesia. The present disclosure is not limited to an EEG application. Alternative systems that utilize a sensor assembly 22 according to the disclosure include electrocardiograph ("ECG" or "EKG") and electromyograph ("EMG") instruments and the like.

The exemplary system 20 shown in FIG. 1 includes a base unit 26, at least one sensor assembly 22, and one or more communication lines 28 that communicatively connect the sensor assembly(ies) 22 with the base unit 26. The communication lines 28 may include electrical conductors configured to conduct electrical signals between sensor assemblies 22 and the base unit 26. In some embodiments, additional hardware including signal amplification devices may be in communication with the communications lines 28. The base unit 26 may include a display device 30, an input device 32, and a system controller 34. Examples of acceptable display devices 30 include LED screens, LCD screens, and the like. Examples of acceptable input devices 32 include a keyboard, a touch screen, a voice commanded unit, or the like.

The system controller 34 is in communication with other system components including the sensor assemblies 22 and the like. The system controller 34 may be in communication with system components to control the operation of the respective component and/or to receive signals from and/or transmit signals to that component to perform the functions described herein. The system controller 34 may include any type of computing device, computational circuit, processor(s), CPU, computer, or the like capable of executing a series of instructions that are stored in memory. The instructions may include an operating system, and/or executable software modules such as program files, system data, buffers, drivers, utilities, and the like. The executable instructions may apply to any functionality described herein to enable the system to accomplish the same algorithmically and/or coordination of system components. The system controller 34 includes or is in communication with one or more memory devices. The present disclosure is not limited to any particular type of memory device, and the memory device may store instructions and/or data in a non-transitory manner. Examples of memory devices that may be used include read-only memory, random access memory, volatile memory, non-volatile memory, static memory, dynamic memory, flash memory, cache memory, and/or any device that stores digital information. The system controller 34 may include, or may be in communication with, an input device that enables a user to enter data and/or instructions, and may include, or be in communication with, an output device configured, for example to display information (e.g., a visual display or a printer), or to transfer data, etc. The example system shown in FIG. 1 has communication lines 28 that connect the sensor assembly(ies) 22 with the base unit 26. In alternative embodiments, the sensor assemblies 22 and base unit 26 / system controller 34 may be configured to communicate via a wireless connection.

Each sensor assembly 22 includes a body 36 and a plurality of electrodes 24. In the embodiment shown in FIG. 2, the sensor assembly 22 includes a body 36 having a main body portion 36A and a discrete body portion 36B connected to one another by a flexible connector 38 that permits the variable positioning between the main body portion 36A and the discrete body portion 36B to suit the application; e.g., the main body portion 36A may be applied to the patient's forehead and the discrete body portion 36B may be applied to the patient's temple region. The flexible connector 38 is configured to provide both physical attachment and electrical communication between the main body portion 36A and the discrete body portion 36B. The present disclosure is not limited to sensor assembly 22 configurations having a main body portion 36A and a discrete body portion 36B; e.g., the sensor body 36 may be a single body or may include more than two body portions.

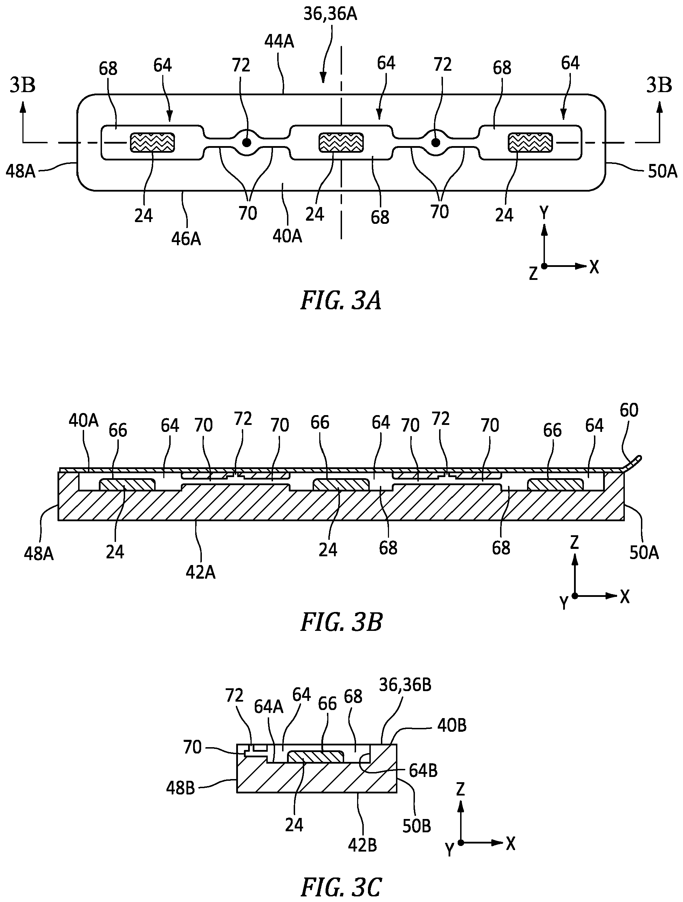

Referring to FIGS. 2, 3A, and 3B, the main body portion 36A includes a contact surface 40A and a back surface 42A. The contact surface 40A and the back surface 42A are on opposite sides of the main body portion 36A. The main body portion 36A example shown in FIGS. 2, 3A, and 3B is generally rectangularly shaped with a contact surface 40A and a back surface 40B that are generally planar. The present disclosure is not limited to a main body portion 36A that is generally rectangularly shaped, and/or having a contact surface 40A and/or a back surface 42A that is generally planar. The main body portion 36A example shown in FIGS. 2, 3A, and 3B includes a first lateral surface 44A, a second lateral surface 46A, a first end surface 48A, and a second end surface 50A. The lateral surfaces 44A, 46A and the end surfaces 48A, 50A may be referred to as "side surfaces". The first lateral surface 44A and the second lateral surface 46A (e.g., extending along an X-axis as shown in the orthogonal axes of FIGS. 3A-3C) are on opposite lateral sides of the main body portion 36A, and the first end surface 48A and the second end surface 50A (e.g., extending along a Y-axis as shown in the orthogonal axes of FIGS. 3A-3C) are on opposite ends of the main body portion 36A. In the example embodiment shown in FIGS. 2, 3A, and 3B, the first and second lateral surfaces 44A, 46A and the first and second end surfaces 48A, 50A extend (e.g., in a Z-axis direction) between the contact surface 40A and the back surface 42A. The main body portion 36A may be described as having a longitudinal axis 52 that extends between the first and second end surfaces 48A, 50A, a widthwise axis 54 that extends between the first and second lateral surfaces 44A, 46A, and a height (or "thickness") that extends between the contact surface 40A and the back surface 42A.

The discrete body portion 36B includes a contact surface 40B and a back surface 42B. The contact surface 40B and the back surface 42B are on opposite sides of the discrete body portion 36B. The discrete body portion 36B example shown in FIGS. 2 and 3C is generally rectangularly shaped with a contact surface 40B and a back surface 42B that are generally planar. The present disclosure is not limited to a discrete body portion 36B that is generally rectangularly shaped, and/or having a contact surface 40B and/or a back surface 42B that is generally planar. The discrete body portion 36B example shown in FIGS. 2 and 3C includes a first lateral surface 44B, a second lateral surface 46B, a first end surface 48B, and a second end surface 50B. The first lateral surface 44B and the second lateral surface 46B (e.g., extending along an X-axis) are on opposite lateral sides of the discrete body portion 36B, and the first end surface 48A and the second end surface 50B (e.g., extending along a Y-axis) are on opposite ends of the discrete body portion 36B. In the example embodiment shown in FIGS. 2 and 3C, the first and second lateral surfaces 44B, 46B and the first and second end surfaces 48B, 50B extend between the contact surface 40B and the back surface 42B . The discrete body portion 36B may be described as having a longitudinal axis 56 that extends between the first and second end surfaces 48B, 50B (e.g., see FIG. 2), and a widthwise axis 58 that extends between the first and second lateral surfaces 44B, 46B and a height (or "thickness") that extends between the contact surface 40B and the back surface 42B.

The present disclosure is not limited to the main body portion 36A and discrete body portion 36B configurations described above.

The main body portion 36A and discrete body portion 36B (or the sensor body 36 as it may be otherwise configured) may be configured in a variety of ways. For example, the aforesaid body portions 36A, 36B may be a unitary body formed from a single material, or may be formed from a plurality of layers that collectively form the respective body 36. In those embodiments that include a unitary body, the body 36 may be configured to contain electrical communication lines extending from the respective electrodes 24 or a printed circuit board (PCB) in communication with the electrodes 24 (detailed below). In those embodiments that include a body portion that includes a plurality of layers, the electrical communication lines or PCB may form, or be disposed in a layer, or be disposed between layers of the body portion.

The body portions 36A, 36B may comprise one or more generally flexible materials (e.g., polymeric materials such as a polyimide). The degree to which the body 36 is flexible can vary depending on the intended application of the sensor assembly 22; e.g., sufficiently flexible to readily correspond to curvature of the application site, etc. In some embodiments, a body portion may comprise a material that is breathable (air and/or moisture) to increase comfort and to facilitate retention of the sensor body 36 on the patient's skin.

In some embodiments, the body portions 36A, 36B may include a removable protection layer 60 (e.g., see FIG. 3B) that is initially disposed in contact with the contact surface 40A, 40B but is intended is to be removed prior to use. The removable protection layer 60 may be adhered to the contact surface 40A, 40B using an adhesive.

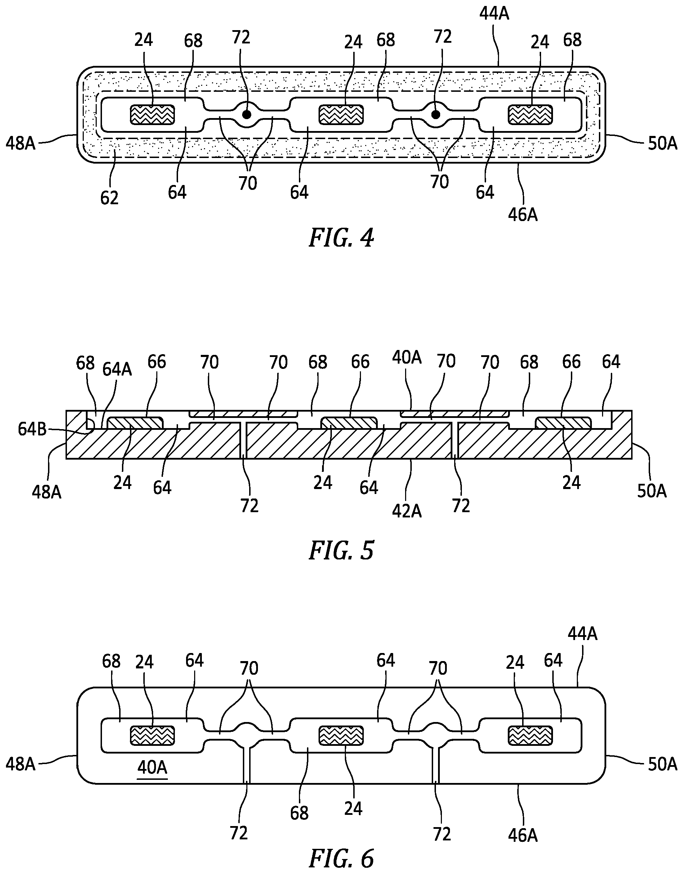

In some embodiments, the body portions 36A, 36B may be configured for attachment to the patient's skin surface via a layer of adhesive 62 disposed on the contact surface 40A, 40B of the respective sensor body portion 36A, 36B. FIG. 4 diagrammatically illustrates an adhesive layer 62 disposed between dashed lines on the contact surface 40A of a sensor main body portion 36A embodiment. The present disclosure is not limited to using adhesive for maintaining contact between the respective body portion 36A, 36B and the patient's skin surface. In alternative embodiments, the respective body portions 36A, 36B may be configured to maintain contact with the patient's skin surface via a suction-cup type element, or may be configured to maintain contact with the patient's skin surface via an independent element such as an elastic member, a bandage member, a strap, a cap, any combination of the same, or other devices for fastening sensors to a patient's body or skin known in the art. The present disclosure is not limited to any particular configuration for maintaining contact between the respective sensor body portion 36A, 36B and the patient's skin surface.

The present disclosure sensor assembly 22 includes at least one electrode 24 and therefore is not limited to any particular number of electrodes 24. The electrode 24 has a sensing surface 66. In the embodiment shown in FIGS. 2 and 3A-3C, the sensor assembly 22 includes there (3) electrodes 24 disposed in the main body portion 36A and a single (1) electrode 24 disposed in the discrete body portion 36B. An example of an acceptable electrode 24 is one made of silver with a coating of silver chloride (Ag/AgCl). The present disclosure is not limited to any particular electrode 24 configuration. In the embodiment shown in FIGS. 2 and 3A-3C, the electrodes 24 in the main body portion 36A are aligned along the longitudinal axis 52 extending between the first and second end surfaces 48A, 50A . In alternative embodiments, electrodes 24 may not be aligned on a common axis. Sensor assembly 22 embodiments may be configured so that certain electrodes 24 operate as a reference electrode 24, and other electrodes 24 operate as an active channel electrode 24. The present disclosure is not limited to any particular operational electrode 24 configuration.

One or more conductive pathways provide the means for electrical signals to travel between the electrodes 24 and the communication line 28 that communicatively connects the sensor assembly 22 with the base unit 26. The conductive pathways may be configured in a variety of different ways. For example, an electrically conductive member (e.g., a copper or gold wire) may electrically connect an electrode 24 with the communication line 28. As another example, the electrodes 24 may be mounted on a printed circuit board (PCB) that is in communication with the communication line 28. The present disclosure is not limited to any particular type of conductive pathway between an electrode 24 and the communication line 28. In some embodiments, present disclosure sensor assembly 22 embodiments may include additional elements such as an amplifier, filters, etc.

Present disclosure sensor assembly 22 embodiments may be configured to use a plurality of channels when the sensor assembly 22 includes a plurality of electrodes 24. For example, the number of channels may be the same as the number of electrodes 24, or the sensor assembly 22 may be configured to use a number of channels that is less than the number of electrodes 24; e.g., four (4) channels and six (6) electrodes 24, etc. The present disclosure sensor assembly 22 is not limited to any particular number of channels.

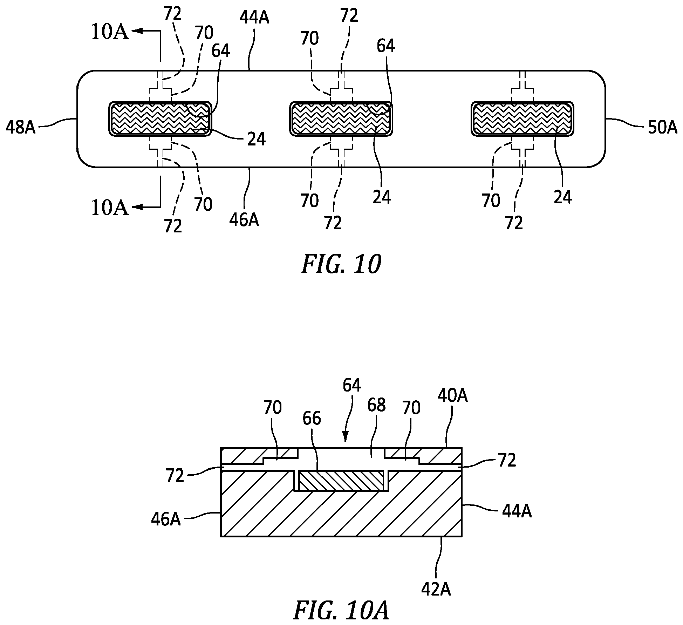

The present disclosure sensor body portions may include a pocket 64 disposed in the contact surface 40A, 40B. An electrode 24 is disposed within each pocket 64. Each pocket 64 has a depth (Z-axis), a length (X-axis), a width (Y-axis). The pockets 64 are open to the contact surface 40A, 40B. In the embodiments shown in FIGS. 2 and 3A-3C, the pockets 64 are shown as generally rectangular and are surrounded by contact surface 40A, 40B. The present disclosure is not limited to generally rectangular pockets 64. The depth of the pockets 64 may be greater than the height of the electrodes 24 to permit a layer of electrolytic gel (detailed herein) to be disposed on the sensing surface 66 of the respective electrode 24. In some embodiments, a pocket 64 may be configured to provide a void 68 around the periphery of the electrode 24, or at least a portion of the periphery; e.g., see embodiments shown in FIGS. 2 and 3A-3C. In these embodiments, the pocket 64 (and the volume defined thereby) may be defined by a base wall 64A of the pocket 64, the sidewalls 64B of the pocket 64, and a plane across the opening of the pocket 64 that is coplanar with the contact surface 40A, 40B. In some embodiments, a pocket 64 may be configured to be substantially contiguous with the periphery of the electrode 24 (except the height); e.g., see embodiment shown in FIGS. 10 and 10A. In these embodiments, the volume of the pocket 64 may be defined by the sensing surface 66 of the electrode 24, the sidewalls 64B of the pocket 64, and a plane across the opening of the pocket 64 that is coplanar with the contact surface 40A, 40B.

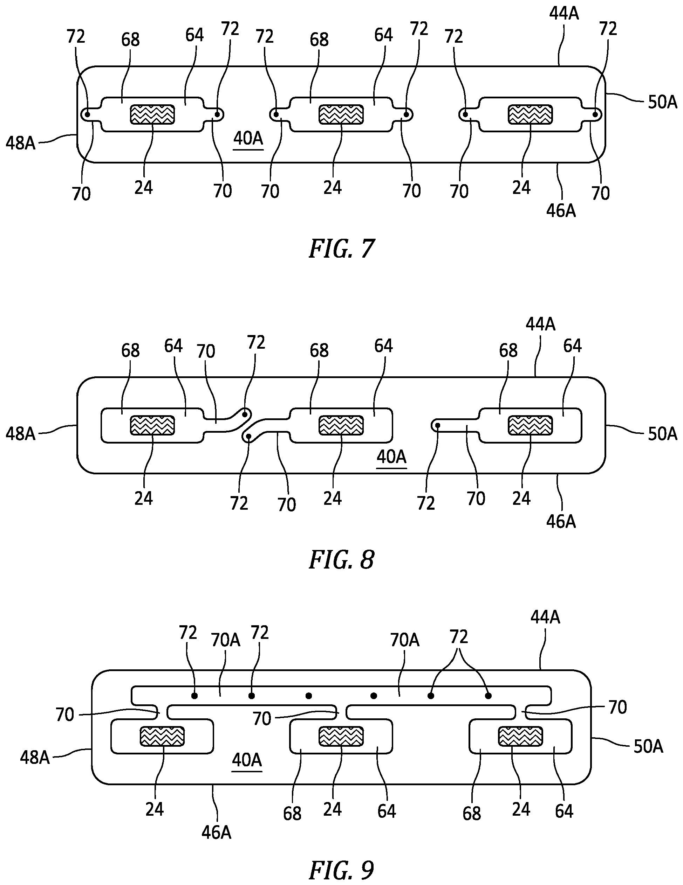

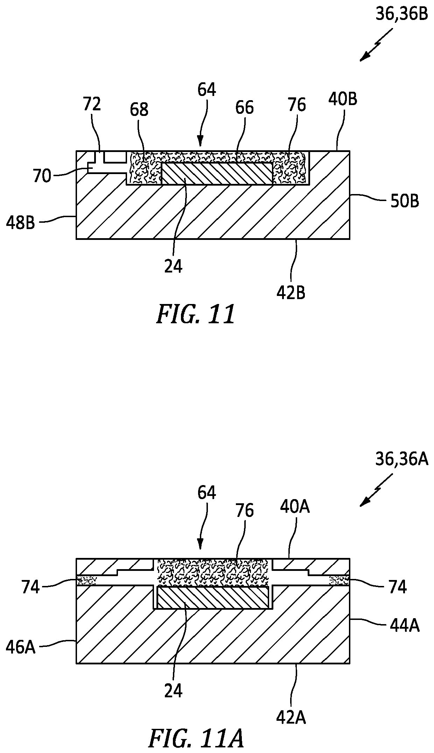

A gel vent is 70 disposed in fluid communication with each pocket 64 and each gel vent 70 includes at least one vent passage 72 that extends between the gel vent 70 and an exterior surface (e.g., 40A, 42A, 44A, 46A, 48A, 50A) of the sensor body 36. In some embodiments, a gel vent 70 extends from the pocket 64 to a distal end; e.g., see FIGS. 7 and 8. In these embodiments, the vent passage 72 may be disposed generally proximate the distal end of the gel vent 70; e.g., to minimize the possibility of electrolytic gel engaging with the vent passage 72. In some embodiments, a first gel vent 70 may extend from a first pocket 64 and connect with a second gel vent 70 in communication with a second pocket 64 (i.e., the first and second gel vents 70 may be described as intersecting at their respective distal ends), and one or more vent passages 72 may be disposed at or near the intersection of the first and second gel vents 70; e.g., see FIG. 3A. In some embodiments, a plurality of gel vents 70 may each extend between a different respective pocket 64 and a common gel vent portion 70A (i.e., the distal end of each gel vent 70 is in fluid communication with the common gel vent portion 70A), and one or more vent passages 72 may be disposed in the common gel vent portion; e.g., see FIG. 9. In the embodiments shown in FIGS. 2 and 3A-3C, the vent passages 72 are shown extending between the respective gel vent 70 and the contact surface 40A, 40B. In an alternative embodiment shown in FIG. 5, the vent passages 72 are shown extending between the respective gel vent 70 and the back surface 42A. In an alternative embodiment shown in FIG. 6, the vent passages 72 are shown extending between the respective gel vent 70 and a lateral surface 46A. The present disclosure is not limited to vent passages 72 in fluid communication with any particular exterior surface (e.g., 40A, 42A, 44A, 46A, 48A, 50A), and may in some embodiments include one or more vent passages 72 in fluid communication with a first exterior surface, and one or more vent passages 72 in fluid communication with a second exterior surface different than the first exterior surface. The gel vents 70 are disposed to provide fluid communication between the pocket 64 in a manner that will permit electrolytic gel to pass between the pocket 64 and the gel vent 70. For example, in those embodiments wherein a pocket 64 provides a void 68 around the periphery of the electrode 24 (e.g., see FIGS. 2 and 3A-3C), a gel vent 70 may be disposed to provide fluid communication between void 68 around the periphery of the electrode 24. In those embodiments wherein a pocket 64 is contiguous with the periphery of the electrode 24 (except the height; e.g., see FIGS. 10 and 10A), a gel vent 70 may be disposed to provide fluid communication between the region of the pocket 64 between the electrode sensing surface 66 and the plane across the opening of the pocket 64 that is coplanar with the contact surface 40A, 40B. If a force is applied on or near the pocket 64 that decreases the volume of the pocket 64, gel may exit the pocket 64 and enter the gel vent 70. Conversely, if the force that decreased the volume of the pocket 64 is no longer applied and the volume of the pocket 64 increases, gel may exit the gel vent 70 and reenter the pocket 64. As will be detailed herein, the present disclosure sensor body 36 embodiments are configured so that the gel vent 70 is initially free of electrolytic gel, or is only partially filled with electrolytic gel. The remainder of the gel vent 70 (including the intersection with the vent passage 72) may be initially filled with air, or may include an air permeable material 74 (e.g., a foam or other porous element - see FIG. 11A) that permits the egress or ingress of air. The air permeable material 74 may be configured to be substantially impermeable to electrolytic gel (e.g., hydrophilic or hydrophobic depending on the type of electrolytic gel) to prevent gel leakage to the exterior of the sensor body 36. In some embodiments, the air permeable material 74 may be configured to be absorbent of the electrolytic gel.

FIGS. 3B, 3C, 5, 10 and 10A diagrammatically illustrate gel vents 70 that are enclosed within the sensor body 36. FIGS. 2, 3A, 4, and 6-9 illustrate the gel vents 70 as solid lines to facilitate the description of the gel vents 70. These gel vents 70 may also be enclosed within the sensor body 36. The present disclosure is not, however, limited to gel vents 70 that are enclosed within the sensor body 36. In alternative embodiments, the gel vents 70 may be configured as open channels disposed in the contact surface 40A, 40B. In those embodiments where a gel vent 70 is configured as an open channel disposed in the contact surface 40A, 40B, a vent passage 72 may extend from the gel vent 70 to an exterior surface other than the contact surface 40A, 40B; e.g., to the back surface 42A, 42B. Gel vents 70 that are enclosed within the sensor body 36 are understood to be preferable, however, to better control electrolytic gel flow between a respective pocket 64 and a gel vent 70 and to avoid potential gel contamination of an adhesive layer 62 (e.g., see FIG. 4) disposed on the contact surface 40A, 40B around the periphery of the pockets 64 for adhering the sensor body 36 to the patient's skin. FIGS. 2, 3A, 3B, 4-6, and 9 illustrate sensor assembly 22 embodiments that include a gel vent 70 in communication with each pocket 64, and gel vents 70 that have one or more common vent passages 72. The embodiment shown in FIG. 9 includes a common gel vent portion 70A that is in communication with a plurality of individual gel vents 70, wherein the common gel vent portion 70A has a plurality of vent passages 72. FIGS. 7 and 10 illustrate sensor assembly 22 embodiments that includes a plurality of gel vents 70 in communication with each pocket 64. FIG. 8 illustrates a sensor assembly 22 embodiment wherein a single gel vent 70 is dedicated to each pocket 64. The embodiments shown in FIGS. 2-10A are non-limiting examples of pocket 64 / gel vent 70 configurations provided to illustrate how the present disclosure may be implemented.

The present disclosure sensor assemblies 22 include an electrolytic gel disposed in communication with the sensing surface 66 of each electrode 24. The electrolytic gel is configured to facilitate electrical signal transmission from patient to the electrode 24; e.g., to improve electrical signal conduction between the patient and the sensing surface 66 of the electrode 24. In those embodiments wherein a pocket 64 provides a void 68 around the periphery of the electrode 24 (or at least a portion of the periphery - e.g., see FIGS. 2, 3A, and 3B), the electrolytic gel may be disposed in the periphery void 68 and the pocket 64 volume between the electrode sensing surface 66 and the plane across the opening of the pocket 64 that is coplanar with the contact surface 40A, 40B. In those embodiments wherein a pocket 64 is substantially contiguous with the periphery of the electrode 24 (except the height - e.g., see FIGS. 10 and 10A), the electrolytic gel may be disposed in the region of the pocket 64 between the electrode sensing surface 66 and the plane across the opening of the pocket 64 that is coplanar with the contact surface. Different gels exhibit different flow properties; e.g., some gels flow more readily than others and may be described as being more or less viscous. In some embodiments, the present disclosure may use a material that may be more appropriately characterized as a fluid rather than a gel. To facilitate the description herein, the term "electrolytic gel" is intended to include electrolytic materials that are known and may be used in with the present disclosure (e.g., to provide an electrical communication path between sensing surface 66 of each electrode 24 and a patient's skin surface), including those electrolytic materials in gel form and fluid form, and the present disclosure is not limited to any particular electrolytic gel.

Referring to FIGS. 11 and 11A, in some embodiments a sensor assembly 22 may include a porous medium 76 such as an open cell foam (e.g., a reticulated foam) disposed within a pocket 64. The porous medium 76 may be disposed in the void 68 around the periphery of the electrode 24, and/or in the region of the pocket 64 between the electrode sensing surface 66 and the plane across the opening of the pocket 64 that is coplanar with the contact surface 40A, 40B. The porous medium 76 may facilitate retention of the electrolytic gel within the pocket 64.

The description of the main body portion 36A and discrete body portion 36B provided herein may be applicable to alternative sensor body 36 configurations (e.g., a single sensor body). In some embodiments, the main body portion 36A may be configured differently from the discrete body portion 36B (e.g., different materials, layers, and the like) or both portions may have the same configuration. Embodiments of the present disclosure are directed to a system 20 having at least one sensor assembly 22 that includes one or more electrodes 24 for sensing the electrical activity of a patient. Embodiments of the present disclosure are also directed to sensor assemblies 22 that includes one or more electrodes 24 for sensing the electrical activity of a patient as described herein.

During use of the present disclosure system 20 and/or sensor assemblies 22, a practitioner (e.g., physician, nurse, etc.) may remove a sensor assembly 22 from its packaging, identify the contact surface 40A, 40B of the sensor body 36 (or body portions), remove the protection layer 60 attached to the contact surface 40A, 40B of the sensor body 36, and position the body 36 for attachment to the patient. For example, in those sensor assembly 22 embodiments that include a main body portion 36A and a discrete body portion 36B, the main body portion 36A may be applied to the patient's forehead and the discrete body portion 36B may be applied to the patient's temple region. Prior to attaching the sensor body 36 to the patient's skin, skin preparation steps (e.g., shaving, cleansing, light abrasion, and the like) may be used to improve the electrical communication between the electrodes 24 and the skin. In those embodiments that include an adhesive layer 62 for attaching the sensor body 36 to the patient's skin, the sensor body 36 may be pressed against the skin to secure attachment. In the event that the force used to press the sensor body 36 against the skin causes electrolytic gel extrusion from a pocket 64, electrolytic gel will pass into the gel vent 70 in communication with the pocket 64. As the electrolytic gel passes into the gel vent 70, air disposed within the gel vent 70 will escape the gel vent 70 to the exterior of the sensor body 36 via the vent passage(s) 54 in communication with the gel vent 70. As the force is released, electrolytic gel may be drawn from the gel vent 70 and back into the pocket 64. As the electrolytic gel is drawn from the gel vent 70 and back into the pocket 64, air from the exterior of the sensor body 36 will be drawn into the respective vent passage 72. In this manner, the potential for electrolytic gel to be forced between the contact surface 40A, 40B of the sensor body 36 and the patient's skin (possible in the absence of a gel vent 70) is mitigated. In those embodiments that include an adhesive layer 62 for attaching a sensor body 36 to the patient's skin, the potential for electrolytic gel to be forced into the adhesive layer 62 (e.g., see FIG. 4) and any potential gel contamination of the adhesive layer 62 is mitigated.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

The singular forms "a," "an," and "the" refer to one or more than one, unless the context clearly dictates otherwise. For example, the term "comprising a sample" includes single or plural samples and is considered equivalent to the phrase "comprising at least one sample." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

As used herein, the terms "comprise", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures-such as alternative materials, structures, configurations, methods, devices, and components, and so on-may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein. For example, in the exemplary embodiments described above within the Detailed Description portion of the present specification, elements may be described as individual units and shown as independent of one another to facilitate the description. In alternative embodiments, such elements may be configured as combined elements. It is further noted that various method or process steps for embodiments of the present disclosure are described herein. The description may present method and/or process steps as a particular sequence. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the description should not be construed as a limitation.

Additionally, even though some features, concepts, or aspects of the disclosures may be described herein as being a preferred arrangement or method, such description is not intended to suggest that such feature is required or necessary unless expressly so stated. Still further, exemplary or representative values and ranges may be included to assist in understanding the present application, however, such values and ranges are not to be construed in a limiting sense and are intended to be critical values or ranges only if so expressly stated.

The treatment techniques, methods, and steps described or suggested herein or in references incorporated herein may be performed on a living animal or on a non-living simulation, such as on a cadaver, cadaver heart, anthropomorphic ghost, or simulator (e.g., with the body parts, or tissue being simulated).

Any of the various systems, devices, apparatuses, etc. in this disclosure may be sterilized (e.g., with heat, radiation, ethylene oxide, hydrogen peroxide) to ensure they are safe for use with patients, and the methods herein may comprise sterilization of the associated system, device, apparatus, etc.; e.g., with heat, radiation, ethylene oxide, hydrogen peroxide.

## Claims

1. A physiological sensor assembly (22), comprising:
at least one electrode (24) having a sensing surface (66); and
a body (36) having a plurality of exterior surfaces (40A, 42A, 44A) including a contact surface (40A), and at least one pocket (64) disposed in the contact surface, wherein the at least one pocket is open to the contact surface and the at least one electrode is disposed in the at least one pocket, and at least one gel vent (70) in fluid communication with the at least one pocket, and at least one vent passage (72) configured to provide fluid communication between the at least one gel vent and at least one said exterior surface.

2. The sensor assembly of claim 1, further comprising an electrolytic gel disposed in the at least one pocket in contact with the sensing surface of the at least one electrode,
optionally wherein the at least one gel vent is configured to receive said electrolytic gel extruded from the at least one pocket, and the at least one vent passage is configured to permit air to escape from the at least one gel vent.

3. The sensor assembly of claim 1 or claim 2, wherein the at least one vent passage is configured to provide fluid communication between the at least one gel vent and the contact surface.

4. The sensor assembly of claim 1 or claim 2, wherein the body exterior surfaces further include a back surface (42A) disposed opposite the contact surface, and the at least one vent passage is configured to provide fluid communication between the at least one gel vent and the back surface.

5. The sensor assembly of claim 1 or claim 2, wherein the body exterior surfaces further include a back surface disposed opposite the contact surface, and at least one side surface (44A, 48A) extending between the back surface and the contact surface around a periphery of the body, and the at least one vent passage is configured to provide fluid communication between the at least one gel vent and the at least one side surface.

6. The sensor assembly of any preceding claim, wherein the at least one pocket is configured to provide a void (68) around at least a portion of a periphery of the at least one electrode, and the at least one gel vent is in fluid communication with the at least a portion of the periphery of the at least one electrode.

7. The sensor assembly of any of claims 1 to 5, wherein the at least one pocket is configured to be substantially contiguous with a periphery of the electrode, and the at least one gel vent is in fluid communication with a portion of the pocket disposed between the sensing surface of the at least one electrode and the contact surface.

8. The sensor assembly of any preceding claim, wherein the at least one pocket includes:
a) a first pocket and a second pocket, and the at least one gel vent includes a first gel vent in fluid communication with the first pocket and a second gel vent in fluid communication with the second pocket, and the first gel vent is independent of the second gel vent;
b) a first pocket and a second pocket, and the at least one gel vent includes a first gel vent in fluid communication with the first pocket and a second gel vent in fluid communication with the second pocket, and the first gel vent is in fluid communication with the second gel vent; or
c) a first pocket and a second pocket, and the at least one gel vent includes a first gel vent in fluid communication with the first pocket and a second gel vent in fluid communication with the second pocket, and the sensor further comprises a common gel vent portion in fluid communication with the first gel vent and the second gel vent, and the at least one vent passage provides fluid communication between the common gel vent portion and the at least one exterior surface.

9. The sensor assembly of any preceding claim, wherein the at least one gel vent and the at least one vent passage are enclosed within the body.

10. The sensor assembly of claim 1, wherein the at least one pocket includes a first pocket, and the at least one gel vent includes a first gel vent and a second gel vent, and the at least one vent passage includes a first vent passage configured to provide fluid communication between the first gel vent and the at least one said exterior surface, and a second vent passage configured to provide fluid communication between the second gel vent and the at least one said exterior surface.

11. The sensor assembly of claim 10, wherein the at least one said exterior surface includes a first exterior surface and a second exterior surface, and the first vent passage is configured to provide fluid communication between the first gel vent and the first exterior surface, and the second vent passage is configured to provide fluid communication between the second gel vent and the second exterior surface.

12. The sensor assembly of any preceding claim, wherein the body includes a main body portion (36A) and a discrete body portion (36B) connected to one another by a flexible connector (38) configured to provide electrical communication between the main body portion and the discrete body portion, and the at least one pocket includes a first pocket and a second pocket disposed in the contact surface of the main body portion and a third pocket disposed in the discrete body portion, and the at least one gel vent includes a first gel vent in fluid communication with the first pocket, a second gel vent in fluid communication with the second pocket, and a third gel vent in fluid communication with the third pocket.

13. The sensor assembly of any preceding claim, wherein the sensor is configured as an electroencephalography ("EEG") sensor.

14. A system (20) for sensing a physiological parameter, comprising:
at least one sensor assembly comprising the physiological sensor assembly (22) of any preceding claim, wherein the at least one electrode is configured to produce first electrical signals representative of second electrical signals sensed from a patient; and
a base unit (26) having a system controller (34) in communication with the at least one electrode of the sensor assembly and a non-transitory memory storing instructions, which instructions when executed cause the system controller to process the first electrical signals to produce information relating to the physiological parameter.

15. The system of claim 14, wherein the at least one sensor assembly is configured as an electroencephalography ("EEG") sensor and the information relating to the physiological parameter includes EEG information.

## Patentansprüche

1. Physiologische Sensoranordnung (22) umfassend:
wenigstens eine Elektrode (24) mit einer Sensorfläche (66); und
einen Körper (36) mit einer Mehrzahl von Außenflächen (40A, 42A, 44A), einschließlich einer Kontaktfläche (40A) und wenigstens einer Tasche (64), die in der Kontaktfläche angeordnet ist, wobei die wenigstens eine Tasche zu der Kontaktfläche offen ist und die wenigstens eine Elektrode in der wenigstens einen Tasche angeordnet ist, und wenigstens eine Gel-Lüftung (70) in Fluidverbindung mit der wenigstens einen Tasche, und wenigstens einen Lüftungskanal (72), der dazu ausgelegt ist, Fluidverbindung zwischen der wenigstens einen Gel-Lüftung und wenigstens einer der Außenflächen bereitzustellen.

2. Sensoranordnung nach Anspruch 1, ferner umfassend ein elektrolytisches Gel, das in der wenigstens einen Tasche in Kontakt mit der Sensorfläche der wenigstens einen Elektrode angeordnet ist,
gegebenenfalls wobei die wenigstens eine Gel-Lüftung dazu ausgelegt ist, das aus der wenigstens einen Tasche extrudierte elektrolytische Gel aufzunehmen, und der wenigstens eine Lüftungskanal dazu ausgelegt ist, zu ermöglichen, dass Luft aus der wenigstens einen Gel-Lüftung entweicht.

3. Sensoranordnung nach Anspruch 1 oder Anspruch 2, wobei der wenigstens eine Lüftungskanal dazu ausgelegt ist, Fluidverbindung zwischen der wenigstens einen Gel-Lüftung und der Kontaktfläche bereitzustellen.

4. Sensoranordnung nach Anspruch 1 oder Anspruch 2, wobei die Körperaußenflächen ferner eine Rückfläche (42A) aufweisen, die gegenüber der Kontaktfläche angeordnet ist, und der wenigstens eine Lüftungskanal dazu ausgelegt ist, Fluidverbindung zwischen der wenigstens einen Gel-Lüftung und der Rückfläche bereitzustellen.

5. Sensoranordnung nach Anspruch 1 oder Anspruch 2, wobei die Körperaußenflächen ferner eine der Kontaktfläche gegenüberliegende Rückfläche und wenigstens eine Seitenfläche (44A, 48A) aufweisen, die zwischen der Rückfläche und der Kontaktfläche um einen Umfang des Körpers verläuft, und der wenigstens eine Lüftungskanal dazu ausgelegt ist, Fluidverbindung zwischen der wenigstens einen Gel-Lüftung und der wenigstens einen Seitenfläche bereitzustellen.

6. Sensoranordnung nach einem der vorstehenden Ansprüche, wobei die wenigstens eine Tasche dazu ausgelegt ist, einen Hohlraum (68) um wenigstens einen Abschnitt eines Umfangs der wenigstens einen Elektrode bereitzustellen, und die wenigstens eine Gel-Lüftung in Fluidverbindung mit dem wenigstens einen Abschnitt des Umfangs der wenigstens einen Elektrode steht.

7. Sensoranordnung nach einem der Ansprüche 1 bis 5, wobei die wenigstens eine Tasche dazu ausgelegt ist, im Wesentlichen an einen Umfang der Elektrode angrenzend zu sein, und die wenigstens eine Gel-Lüftung in Fluidverbindung mit einem Abschnitt der Tasche steht, der zwischen der Sensorfläche der wenigstens einen Elektrode und der Kontaktfläche angeordnet ist.

8. Sensoranordnung nach einem der vorstehenden Ansprüche, wobei die wenigstens eine Tasche aufweist:
a) eine erste Tasche und eine zweite Tasche, und wobei die wenigstens eine Gel-Lüftung eine erste Gel-Lüftung in Fluidkommunikation mit der ersten Tasche und eine zweite Gel-Lüftung in Fluidkommunikation mit der zweiten Tasche aufweist und die erste Gel-Lüftung unabhängig von der zweiten Gel-Lüftung ist;
b) eine erste Tasche und eine zweite Tasche, und wobei die wenigstens eine Gel-Lüftung eine erste Gel-Lüftung in Fluidverbindung mit der ersten Tasche und eine zweite Gel-Lüftung in Fluidverbindung mit der zweiten Tasche aufweist und die erste Gel-Lüftung in Fluidverbindung mit der zweiten Gel-Lüftung steht; oder
c) eine erste Tasche und eine zweite Tasche, und wobei die wenigstens eine Gel-Lüftung eine erste Gel-Lüftung in Fluidverbindung mit der ersten Tasche und eine zweite Gel-Lüftung in Fluidverbindung mit der zweiten Tasche aufweist und der Sensor ferner einen gemeinsamen Gel-Lüftungsabschnitt in Fluidverbindung mit der ersten Gel-Lüftung und der zweiten Gel-Lüftung umfasst und der wenigstens eine Lüftungskanal Fluidverbindung zwischen dem gemeinsamen Gel-Lüftungsabschnitt und der wenigstens einen Außenfläche bereitstellt.

9. Sensoranordnung nach einem der vorstehenden Ansprüche, wobei die wenigstens eine Gel-Lüftung und der wenigstens eine Lüftungskanal innerhalb des Körpers eingeschlossen sind.

10. Sensoranordnung nach Anspruch 1, wobei die wenigstens eine Tasche eine erste Tasche aufweist und die wenigstens eine Gel-Lüftung eine erste Gel-Lüftung und eine zweite Gel-Lüftung aufweist und der wenigstens eine Lüftungskanal einen ersten Lüftungskanal aufweist, der dazu ausgelegt ist, Fluidverbindung zwischen der ersten Gel-Lüftung und der wenigstens einen Außenfläche bereitzustellen, und einen zweiten Lüftungskanal, der dazu ausgelegt ist, Fluidverbindung zwischen der zweiten Gel-Lüftung und der wenigstens einen der Außenflächen bereitzustellen.

11. Sensoranordnung nach Anspruch 10, wobei die wenigstens eine Außenfläche eine erste Außenfläche und eine zweite Außenfläche aufweist und der erste Lüftungskanal dazu ausgelegt ist, Fluidverbindung zwischen der ersten Gel-Lüftung und der ersten Außenfläche bereitzustellen, und der zweite Lüftungskanal dazu ausgelegt ist, Fluidverbindung zwischen der zweiten Gel-Lüftung und der zweiten Außenfläche bereitzustellen.

12. Sensoranordnung nach einem der vorstehenden Ansprüche, wobei der Körper einen Hauptkörperabschnitt (36A) und einen diskreten Körperabschnitt (36B) aufweist, die durch einen flexiblen Verbinder (38) miteinander verbunden sind, der dazu ausgelegt ist, elektrische Kommunikation zwischen dem Hauptkörperabschnitt und dem diskreten Körperabschnitt bereitzustellen, und die wenigstens eine Tasche eine erste Tasche und eine zweite Tasche aufweist, die in der Kontaktfläche des Hauptkörperabschnitts angeordnet sind, und eine dritte Tasche, die in dem diskreten Körperabschnitt angeordnet ist, und die wenigstens eine Gel-Lüftung eine erste Gel-Lüftung in Fluidverbindung mit der ersten Tasche, eine zweite Gel-Lüftung in Fluidverbindung mit der zweiten Tasche und eine dritte Gel-Lüftung in Fluidverbindung mit der dritten Tasche aufweist.

13. Sensoranordnung nach einem der vorstehenden Ansprüche, wobei der Sensor als Elektroenzephalographie("EEG")-Sensor ausgelegt ist.

14. System (20) zum Erfassen eines physiologischen Parameters, umfassend:
wenigstens eine Sensoranordnung, die die physiologische Sensoranordnung (22) nach einem der vorstehenden Ansprüche umfasst, wobei die wenigstens eine Elektrode dazu ausgelegt ist, erste elektrische Signale zu erzeugen, die zweite elektrische Signale darstellen, die von einem Patienten erfasst werden; und
eine Basiseinheit (26) mit einer Systemsteuerung (34) in Kommunikation mit der wenigstens einen Elektrode der Sensoranordnung und einem nichtflüchtigen Speicher, der Anweisungen speichert, wobei die Anweisungen bei Ausführung die Systemsteuerung veranlassen, die ersten elektrischen Signale zu verarbeiten, um Informationen bezüglich des physiologischen Parameters zu erzeugen.

15. System nach Anspruch 14, wobei die wenigstens eine Sensoranordnung als Elektroenzephalographie("EEG")-Sensor ausgelegt ist und die Informationen bezüglich des physiologischen Parameters EEG-Informationen enthalten.

## Revendications

1. Ensemble capteur physiologique (22), comprenant :
au moins une électrode (24) ayant une surface de détection (66) ; et
un corps (36) comportant une pluralité de surfaces extérieures (40A, 42A, 44A) comportant une surface de contact (40A), et au moins une poche (64) disposée dans la surface de contact, l'au moins une poche étant ouverte sur la surface de contact et l'au moins une électrode étant disposée dans l'au moins une poche, et au moins une évacuation de gel (70) en communication de fluide avec l'au moins une poche, et au moins un passage d'évacuation (72) configuré pour assurer une communication de fluide entre l'au moins une évacuation de gel et au moins une dite surface extérieure.

2. Ensemble capteur selon la revendication 1, comprenant en outre un gel électrolytique disposé dans l'au moins une poche en contact avec la surface de détection de l'au moins une électrode,
éventuellement dans lequel l'au moins une évacuation de gel est configurée pour recevoir ledit gel électrolytique extrudé en provenance de l'au moins une poche, et l'au moins un passage d'évacuation est configuré pour permettre à l'air de s'échapper de l'au moins une évacuation de gel.

3. Ensemble capteur selon la revendication 1 ou la revendication 2, dans lequel l'au moins un passage d'évacuation est configuré pour assurer une communication fluidique entre l'au moins une évacuation de gel et la surface de contact.

4. Ensemble capteur selon la revendication 1 ou la revendication 2, dans lequel les surfaces extérieures du corps comportent en outre une surface arrière (42A) disposée en regard de la surface de contact, et l'au moins un passage d'évacuation est configuré pour assurer une communication fluidique entre l'au moins une évacuation de gel et la surface arrière.

5. Ensemble capteur selon la revendication 1 ou la revendication 2, dans lequel les surfaces extérieures du corps comportent en outre une surface arrière disposée en regard de la surface de contact, et au moins une surface latérale (44A, 48A) s'étendant entre la surface arrière et la surface de contact autour d'une périphérie du corps, et l'au moins un passage d'évacuation est configuré pour assurer une communication fluidique entre l'au moins une évacuation de gel et l'au moins une surface latérale.

6. Ensemble capteur selon une quelconque revendication précédente, dans lequel l'au moins une poche est configurée pour fournir un vide (68) autour d'au moins une partie d'une périphérie de l'au moins une électrode, et l'au moins une évacuation de gel est en communication fluidique avec l'au moins une partie de la périphérie de l'au moins une électrode.

7. Ensemble capteur selon l'une quelconque des revendications 1 à 5, dans lequel l'au moins une poche est configurée pour être sensiblement contiguë à une périphérie de l'électrode, et l'au moins une évacuation de gel est en communication fluidique avec une partie de la poche disposée entre la surface de détection de l'au moins une électrode et la surface de contact.

8. Ensemble capteur selon une quelconque revendication précédente, dans lequel l'au moins une poche comporte :
a) une première poche et une deuxième poche, et l'au moins une évacuation de gel comporte une première évacuation de gel en communication fluidique avec la première poche et une deuxième évacuation de gel en communication fluidique avec la deuxième poche, et la première évacuation de gel est indépendante de la deuxième évacuation de gel ;
b) une première poche et une deuxième poche, et l'au moins une évacuation de gel comporte une première évacuation de gel en communication fluidique avec la première poche et une deuxième évacuation de gel en communication fluidique avec la deuxième poche, et la première évacuation de gel est en communication fluidique avec la deuxième évacuation de gel ; ou
c) une première poche et une deuxième poche, et l'au moins une évacuation de gel comporte une première évacuation de gel en communication fluidique avec la première poche et une deuxième évacuation de gel en communication fluidique avec la deuxième poche, et le capteur comprend en outre une partie évacuation de gel commune en communication fluidique avec la première évacuation de gel et la deuxième évacuation de gel, et l'au moins un passage d'évacuation assure une communication fluidique entre la partie évacuation de gel commune et l'au moins une surface extérieure.

9. Ensemble capteur selon une quelconque revendication précédente, dans lequel l'au moins une évacuation de gel et l'au moins un passage d'évacuation sont enfermés à l'intérieur du corps.

10. Ensemble capteur selon la revendication 1, dans lequel l'au moins une poche comporte une première poche, et l'au moins une évacuation de gel comporte une première évacuation de gel et une deuxième évacuation de gel, et l'au moins un passage d'évacuation comporte un premier passage d'évacuation configuré pour assurer une communication fluidique entre la première évacuation de gel et l'au moins une dite surface extérieure, et un second passage d'évacuation configuré pour assurer une communication fluidique entre la deuxième évacuation de gel et l'au moins une dite surface extérieure.

11. Ensemble capteur selon la revendication 10, dans lequel l'au moins une dite surface extérieure comporte une première surface extérieure et une deuxième surface extérieure, et le premier passage d'évacuation est configuré pour assurer une communication fluidique entre la première évacuation de gel et la première surface extérieure, et le deuxième passage d'évacuation est configuré pour assurer une communication fluidique entre la deuxième évacuation de gel et la seconde surface extérieure.

12. Ensemble capteur selon une quelconque revendication précédente, dans lequel le corps comporte une partie de corps principale (36A) et une partie de corps discrète (36B) reliées l'une à l'autre par un raccord flexible (38) configuré pour assurer une communication électrique entre la partie de corps principale et la partie de corps discrète, et l'au moins une poche comporte une première poche et une deuxième poche disposées dans la surface de contact de la partie de corps principale et une troisième poche disposée dans la partie de corps discrète, et l'au moins une évacuation de gel comporte une première évacuation de gel en communication fluidique avec la première poche, une deuxième évacuation de gel en communication fluidique avec la deuxième poche, et une troisième évacuation de gel en communication fluidique avec la troisième poche.

13. Ensemble capteur selon une quelconque revendication précédente, dans lequel le capteur est configuré en tant que capteur d'électroencéphalographie (« EEG »).

14. Système (20) permettant de détecter un paramètre physiologique, comprenant :
au moins un ensemble capteur comprenant l'ensemble capteur physiologique (22) selon une quelconque revendication précédente, dans lequel l'au moins une électrode est configurée pour produire des premiers signaux électriques représentatifs de seconds signaux électriques détectés à partir d'un patient ; et
une unité de base (26) présentant un dispositif de commande système (34) en communication avec l'au moins une électrode de l'ensemble capteur et une mémoire non transitoire stockant des instructions, lesquelles instructions, lorsqu'elles sont exécutées, amènent le dispositif de commande système à traiter les premiers signaux électriques afin de produire des informations relatives au paramètre physiologique.

15. Système selon la revendication 14, dans lequel l'au moins un ensemble capteur est configuré en tant que capteur d'électroencéphalographie (« EEG ») et les informations relatives au paramètre physiologique comportent des informations EEG.
